# EUROPEAN PATENT APPLICATION

(11) **EP 1 640 358 A1**
(43) Date of publication of application: **29.03.2006**
(21) Application number: 04021896.8
(22) Date of filing: 15.09.2004
(51) Int. Cl.: C07C 209/26, C07C 209/28, C07C 211/27

(54) **Synthesis of amine stereoisomers**

(71) Applicant: INTERNATIONAL UNIVERSITY BREMEN GMBH, 28759 Bremen (DE)
(72) Inventor: Nugent, Thomas Christopher, 28759 Bremen (DE); Ghosh, Abhijit K., 28759 Bremen (DE); Wakchaure, Vijay N., 28759 Bremen (DE); Mohanty, Rashmi R. c/o Internat. University Bremen, 28759 Bremen (DE)
(74) Representative: Eisenführ, Speiser & Partner

(57) **Abstract**

The invention relates to methods for producing secondary and tertiary amine diastereomers and corresponding enantioenriched primary or secondary chiral amine products.

## Description

The invention relates to methods for producing secondary and tertiary amine diastereomers and their corresponding primary or secondary chiral amine enantioenriched products.

The synthesis of amines is commonly achieved through the manipulation of ketones. Two types of processes have been established: The ketone can be directly converted to an amine in a reductive amination reaction. This reaction has the advantage of being a one-step reaction, it eliminates the need to extract and purify any intermediates. The frequently applied alternative process is a two-step process. In the first step, a ketone is reacted with an amine to give an imine. Said imine is isolated and further reduced in a second step to provide the desired amine.

All references to amine nitrogen atoms relate exclusively to the nitrogen atom that is introduced into a ketone educt to yield a respective amine, unless otherwise indicated. All substances referred to hereinafter may comprise further nitrogen atoms or further amine moieties.

Within the present invention, the term "aliphatic ketone" educt denotes a ketone educt wherein the carbon atom of the carboxy group that is converted into an amine group is not directly covalently connected to an atom of an aromatic or heteroaromatic moiety. Aliphatic ketones according to the invention therefore may comprise one or more aromatic moieties, provided that, for each aromatic moiety, the shortest chain of atoms covalently linking an atom of the aromatic moiety to the carbon atom of the carboxy group is of at least one or more atoms in length. An "aromatic ketone" educt according to the invention denotes a ketone educt wherein the carbon atom of the carboxy group is directly covalently connected to one or more aromatic or heteroaromatic moieties.

Similarly, a reference to an "aliphatic amine" product denotes an amine product obtained from an aliphatic ketone educt according to the invention; whereas the term "aromatic amine" product denotes an amine product obtained from an aromatic ketone according to the invention.

The ketone educt may contain a chiral centre. According to the invention, the maximum value of the method is generally realized when a prochiral ketone is used. The term "prochiral ketone" denotes a ketone educt wherein the carbon atom of the carboxy group is not directly covalently connected to a chiral centre. Accordingly, a prochiral ketone useful within the method of the present invention may have one or more chiral centre(s), as long as it/they is/are not directly covalently connected to the carbon atom of the carboxy group that is to be turned into an amino group. Preferably, the prochiral ketone is devoid of any chiral centre.

Within the present invention, all references to stereoisomers (diastereomers and enantiomers) relate exclusively to a) the newly generated chiral centre of the former ketone educt carboxy group, or b) the chiral centre(s) of the respective amine nitrogen atom of the chiral auxiliary, unless otherwise indicated. For example, a compound will be regarded as being enantiopure for the purposes of the present invention if the chiral centre(s) adjacent to the amino nitrogen atom has/have the same stereoconfiguration for essentially all molecules of that compound, regardless of any further chiral centres comprised in said compound.

Clifton et al. (J. Med. Chem., 1982, 670-679) disclose a method for producing [R-(R*, R*)]-α-Methyl-N-(1-phenylethyl)benzenepropanamine by reacting (R)-(+)-α-Methylbenzylamine and benzylacetone under hydrogenating conditions in the presence of Raney nickel. They achieved a crude yield (before purification) of 91% with a diastereomeric ratio of 90:10 (RR/SR). After recrystallisation the highly enriched RR diastereoisomer was obtained in 37% yield. Furthermore, they disclose a method for producing the corresponding primary amine R-α-Methylbenzenepropanamine by hydrogenolysis of the highly enriched RR diastereomer [R-(R*, R*)]-α-Methyl-N-(1-phenylethyl)benzenepropanamine over 10% PdO/C. The primary amine is produced in 70% yield and in >99% enantiomeric purity.

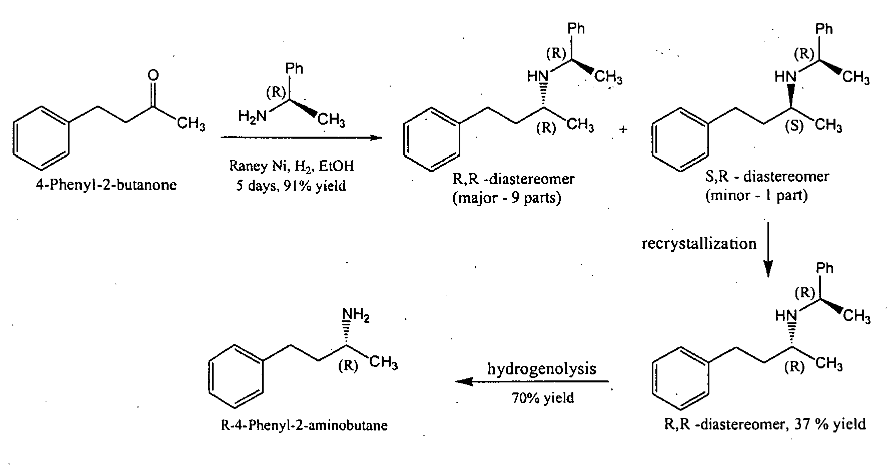

However, the reaction of (R)-(+)-α-Methylbenzylamine and benzylacetone (4-phenyl-2-butanone) is very slow and requires 5 days (120 h) to yield sufficient quantities of the secondary amine.

Alexakis et al. (Tetrahedron Letters, 2004, 1449-1451) disclose a method for producing C₂-symmetrical aromatic secondary amines by reacting an aromatic ketone and different enantiopure aromatic primary amines with an effective amount of Ti(OiPr)₄/H₂/Pd-C. However, the reported isolated yields of 51 % and 63 % are comparatively low. The method is not adaptable for the production of aliphatic primary amine enantiomers.

It was therefore a problem of the present invention to provide a method for producing a secondary or tertiary amine diastereomer, wherein the method should allow for the production of secondary or tertiary amine diastereomers with a diastereomeric excess of ≥70 %. The method should also be fast, preferably achieving a secondary amine diastereomer yield of 70 % with a diastereomeric excess of ≥70 % within 48 h of reaction time and should not rely on reaction pressures higher than 50 bar or reaction temperatures higher than 50 °C.

A further object of the invention was to provide a method for producing enantiomerically pure or enantiomerically enriched primary or secondary amine products. The method should also be useful for producing enantiopure or enantioenriched aliphatic primary or secondary amine products.

The object of the invention is accomplished by a method for producing a secondary or tertiary amine diastereomer, comprising or consisting of the step of reductively aminating a preferably prochiral ketone with a primary or secondary chiral amine auxiliary to produce the respective secondary or tertiary amine diastereomer, wherein
- the reductive amination is effected in the presence of a hydride,
- optionally with the removal of water, and wherein
- the reductive amination is performed under the influence of a mild Lewis acid.

This method allows the production of secondary or tertiary amine diastereomers with a high diastereomeric excess (de) in a fast reaction with a high yield and under reaction pressures of less than 50 bar and reaction temperatures of less than 50 °C. The reaction parameters, in particular the meaning of the term "mild Lewis acid", will be described hereinafter with reference to a second, preferred teaching according to the invention. The advantages conveyed by the method according to said second teaching of the invention can also be achieved by the method according to the invention just described above, but generally to a lesser extent.

The hydrides according to the aforementioned method preferably are: Sodium borohydride, zinc borohydride, sodium cyanoborohydride, lithium aminoborohydrides, lithium aluminum hydride, diisobutylaluminum hydride, trialkoxysilanes, for example triethoxysilane.

According to the second, and preferred teaching of the invention, there is provided a method for producing a secondary or tertiary amine diastereomer, said method comprising or consisting of the step of reductively aminating a (preferably prochiral) ketone with a primary or secondary chiral amine auxiliary to produce the respective secondary or tertiary amine diastereomer, wherein
- the reductive amination is effected in the presence of a hydrogenating catalyst and a hydrogenating agent,
- optionally with the removal of water, and wherein
- the hydrogenating catalyst is a metal catalyst selected from the group consisting of nickel, copper, iron, cobalt, ruthenium, rhodium, palladium, osmium, iridium and platinum metal catalysts or mixtures thereof, and wherein
- the reductive amination is performed under the influence of a mild Lewis acid.

This preferred method can generally be described by the following reaction scheme: 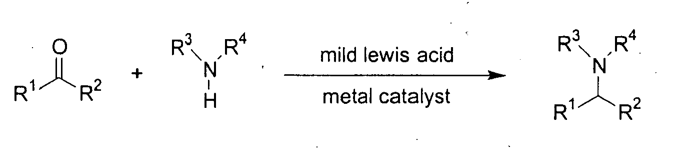 wherein each moiety R1, R2, R3 and R4 is, independent from any other moiety R1, R2, R3 and R4, alkyl, alkenyl, alkynyl, aryl, heteroalkyl, heteroalkenyl, heteroalkynyl, heteroaryl, alkylaryl, alkenylaryl, alkynylaryl, heteroalkylaryl, heteroalkenylaryl, heteroalkynylaryl, arylalkyl, arylalkenyl, arylalkynyl, heteroarylalkyl, heteroarylalkenyl, heteroarylalkynyl, and wherein R3 can also be hydrogen, and wherein at least one of R3 and R4 comprises a chiral centre, and wherein R1 and R2 may together form a ring and wherein R3 and R4 may together form a ring. Preferred ketones and amine auxiliaries are described in greater detail below.

It has now been found that by combining the effects of a mild Lewis acid and a metal catalyst of the type described above, secondary or tertiary amine diastereomers can be produced with a high diastereomeric excess in a fast reaction (typically 24 h or less) with a high yield and under reaction pressures of less than 50 bar and reaction temperatures of less than 50 °C (see examples). Unless stated otherwise, all references hereinafter to a method according to the invention (for producing a secondary or tertiary amine diastereomer from a ketone) relate particularly to the method according to the second, preferred teaching of the invention.

When compared to the method according to the first teaching of the invention, the method according to the second, preferred teaching of the invention is particularly advantageous because hydrides generally impart a lower de on the secondary or tertiary chiral amine diastereomer, generate excessive amounts of waste compared to the preferred method, cannot be used in as many different solvents as the prefered method and in some instances are toxic and/or are price restrictive.

Within the present invention, a "good diastereoselectivity" is one with a diastereomeric excess of ≥70 %. The present invention generally allows a diastereomeric excess of ≥70 % to be achieved.

The chiral amine auxiliary is a primary or secondary amine auxiliary in substantially enantiopure form or, preferably, with an enantiomeric excess or diastereomeric excess of ≥70 %. The chiral amine auxiliary either comprises at least one centre of chirality covalently connected to the amino nitrogen atom that is to react with the carboxy group of the ketone educt by a chain of at most 6 atoms, preferably at most 3 atoms, and preferably is directly covalently connected thereto. The chiral amine auxiliary may also or alternatively comprise a chiral axis or plane which like a chiral centre is/are capable of stereo-differentation and thus is/are able to influence the enantiomeric excess at the new chiral centre where the carboxy group of the ketone educt used to reside and therefore the diastereomeric excess of the secondary or tertiary chiral amine produced.

Water is optionally removed from the reductive amination reaction, typically by entrapping it with a molecular sieve, a zeolite and/or a dehydrating agent like MgSO₄. Water removal (or trapping) as described just before will typically enhance the reaction rate, so that a faster reaction time is common under otherwise identical reaction conditions. Reaction times of 6 h to 13 h can be achieved when the reductive amination according to the invention is performed in the presence of an effective amount of a molecular sieve or zeolite.

The mild Lewis acid is preferably not a proton source. Preferably, the mild Lewis acid is selected from the group consisting
a) a metal alkoxide, in particular
   - a metal isopropoxide, in particular an isopropoxide of titanium, aluminium, zirconium, vanadium, niobium, cerium, samarium, scandium, iron,
   - a metal methoxide or metal ethoxide, in particular titanium methoxide and iron ethoxide,
b) a metal acetate, in particular, scandium acetate and silver acetate,
c) a metal halogenide, in particular iron trichloride, scandium trichloride, zinc bromide, titanium tetrachloride, aluminium trichloride, zirconium tetrachloride or a chloride or bromide of vanadium, niobium, cerium, samarium, scandium,
d) a metal amine, in particular an amide of titanium, aluminium, zirconium, vanadium, niobium, cerium, samarium, scandium, iron,
e) a silane, in particular chlorotrimethylsilane, phenyltrichlorosilane, phenyltriethoxysilane,
f) a borate, in particular trimethyl borate, triisopropylborate, boron amide, boron trifluoride etherate, boron trifuoride,
g) a heteropolar compound, in particular a sulfonate of titanium, aluminium, zirconium, vanadium, niobium, cerium, samarium, scandium, iron, boron,
or a mixture of two or more members of the group. It is particularly preferred if the mild Lewis acid comprises an element in the respective oxidation state selected from the group consisting of Ti (IV), Al (III), Zr (IV), Vn (V), Nb (V), Ce (IV), Sm (III), Sc (III), Fe (III), B (III). These mild Lewis acids are particularly suitable to be used in a method according to the invention.

According to the invention, the mild Lewis acid is preferably one comprising titanium, aluminium, zirconium and/or boron. These mild Lewis acids have resulted in very good product yields (≥70 %) under fast reaction times, typically 6 h to 16 h, and with good diastereoselectivity, particularly in a method according to the second, preferred, teaching of the invention.

In a particularly preferred method according to the present invention the mild Lewis acid is a metal alkoxide or boron-based Lewis acid. Best results have so far been achieved by employing titanium isopropoxide, aluminium isopropoxide, zirconium isopropoxide, trimethylborate, triisopropylborate, and boron trifluoride (which is commonly available in the form of the respective diethylether complex), of which titanium isopropoxide is particularly preferred because it allows moderately faster reactions compared to aluminium isopropoxide and zirconium isopropoxide. According to the invention, titanium isopropoxide, aluminium isopropoxide and the borates and boron trifluoride are generally preferred over zirconium isopropoxide because of their price.

The metal catalyst preferably is a nickel, palladium, platinum and/or copper metal catalyst, and preferably in a Raney metal catalyst form. High product yields and good diastereomeric excess values have been achieved by using a nickel metal catalyst in a method according to the present invention. It is particularly preferred when the nickel metal catalyst is a powder with a median particle diameter of 2 mm or less. Most preferred is a method according to the invention, wherein the metal catalyst is Raney nickel. This catalyst has so far resulted in the highest product yields and diastereomeric excesses in a method according to the invention (see examples).

The activity of the Raney nickel catalyst can generally be increased by addition of small amounts of triethylamine hexachloroplatinate (IV) or by small amounts of aluminium resident in the catalyst. Furthermore, the diastereoselectivity of the method according to the invention can generally be enhanced when the Raney catalyst, in particular Raney nickel, has attached thereto an enantiopure or enantioenriched (de or ee, respectively, ≥70 %) compound, e.g. an enantiopure or enantioenriched acid or ester, e.g. tartaric acid.

The method according to the present invention preferably comprises the step of forming a pre-reaction mixture by mixing the starting materials and reagents in the following order:
(1) solvent (if applicable),
(2) ketone, preferably a prochiral ketone,
(3) mild Lewis acid, preferably titanium isopropoxide, aluminium isopropoxide, zirconium isopropoxide and/or boron trifluoride,
(4) chiral amine auxiliary

Then, after formation of the pre-reaction mixture, the metal catalyst is added and hydrogenation is initiated.

According to the invention, at the initiation of hydrogenation, the preferably prochiral ketone and the chiral amine auxiliary are preferably present in the reaction mixture in a molar ratio of 1:4 to 4:1 and particularly preferred in an equimolar amount. Furthermore, the chiral amine auxiliary and/or the (prochiral) ketone on the one hand and the mild Lewis acid on the other hand are preferably present in the reaction mixture (at the initiation of hydrogenation) in a molar ratio of 1:4 to 4:1, preferably in an equimolar amount. It is particularly preferred that the (prochiral) ketone, the chiral amine auxiliary and the mild Lewis acid are present in the reaction mixture in equimolar amounts. These preferred ratios aid the purification of the secondary or tertiary amine diastereomer product, reduce the cost of the process, and the waste produced, while providing good reaction yields and good diastereoselectivity for the chiral amine product as taught by the second and preferred teaching of the invention .

The metal catalyst, in particular Raney nickel, is preferably added to said pre-reaction mixture 5 min to 6 h after formation of said pre-reaction mixture with simultaneous initiation of hydrogenation. It is particularly preferred to add the metal catalyst, particularly Raney nickel, and then initiate hydrogenation within 0-60 min after addition of the said metal catalyst. In some instances, diastereomeric excesses (des) are slightly improved if the reaction mixture is pre-stirred for 30 min to 2 h before adding the metal catalyst, particularly Raney nickel, and initiating hydrogenation by pressurising the reaction mixture with hydrogen gas.

The chiral primary amine used in the reductive amination reaction is enantiopure (with respect to the chiral centre adjacent to the amino nitrogen atom) or is present in the reaction in an enantiomeric excess (ee) of ≥70%, preferably ≥90% and most preferred ≥95%. In general, the greater the enantiomeric excess of the chiral primary amine, the greater the diastereomeric excess of the desired secondary or tertiary amine diastereomer produced.

The chiral auxiliary preferably is one of the formula 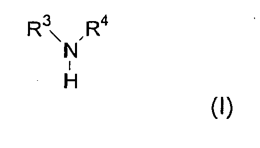 wherein
- R3 and R4: are, independent from one another, alkyl, alkenyl, alkynyl, aryl, heteroalkyl, heteroalkenyl, heteroalkynyl, heteroaryl, alkylaryl, alkenylaryl, alkynylaryl, heteroalkylaryl, heteroalkenylaryl, heteroalkynylaryl, arylalkyl, arylalkenyl, arylalkynyl, heteroarylalkyl, heteroarylalkenyl, heteroarylalkynyl, and wherein R3 can also be hydrogen, and wherein
moieties R3 and R4 may together form a ring, and wherein
at least one of R3 and R4 comprises a chiral centre. Preferably, each moiety R3 and R4 comprises at most 30 carbon atoms.

Particularly preferred is a method according to the present invention wherein the chiral amine auxiliary is a chiral primary amine auxiliary, such that R3 is H.

A particularly preferred chiral primary amine auxiliary is (R)-1-methylbenzylamine. Another particularly preferred chiral primary amine auxiliary is (S)-1-methylbenzylamine. These amines are readily obtainable in enantiopure form or with an enantiomeric excess (ee) of ≥70% (technical grade quantities). Also, the part of the chiral amine auxiliary that is not generally a constitutive part of the amine product can be easily cleaved by hydrogenolysis to produce a substantially enantiopure or enantioenriched primary or secondary amine product.

Further useful chiral auxiliaries are those with a chiral axis or chiral plane. Of these groups, particularly preferred chiral auxiliaries are 2,2'-diamino-6,6'-dimethyl-1,1'-biphenyl and 2, 2'-diamino-1,1'-binaphtyl.

It is furthermore particularly preferred if the ketone is of the form 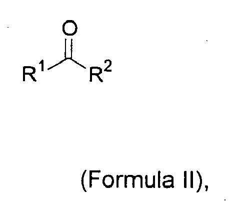 wherein moieties R1and R2 are, independent from one another,
a) alkyl, alkenyl, alkynyl, aryl, heteroalkyl, heteroalkenyl, heteroalkynyl, heteroaryl, alkylaryl, alkenylaryl, alkynylaryl, heteroalkylaryl, heteroalkenylaryl, heteroalkynylaryl, arylalkyl, arylalkenyl, arylalkynyl, heteroarylalkyl, heteroarylalkenyl, heteroarylalkynyl
b) and wherein moieties R1 and R2 may together form a ring.

Preferably, each moiety R1 and R2 comprises at most 30 carbon atoms.

Furthermore, it is preferred that the ketone is prochiral. It is also preferred that the (preferably prochiral) ketone is an aliphatic ketone, preferably an aliphatic prochiral ketone. Synthesis of the corresponding secondary or tertiary amine diastereomers and subsequent cleavage of the chiral auxiliary produces enantiopure or enantioenriched primary or secondary amine products respectively, so far this has been difficult to achieve. The method according to the invention advantageously allows production of such secondary or tertiary amines and the corresponding primary or secondary amine product enantiomers in a fast reaction, typically in less than 16 h, at a temperature below 50 °C and a pressure of less than 50 bar. These advantages particularly apply to those methods according to the invention that use prochiral ketones, particularly aliphatic prochiral ketones.

Particularly preferred generic and specific examples of ketones are:

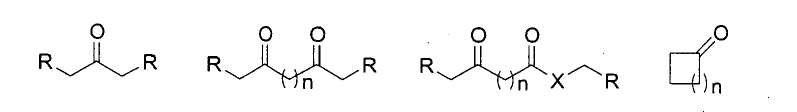

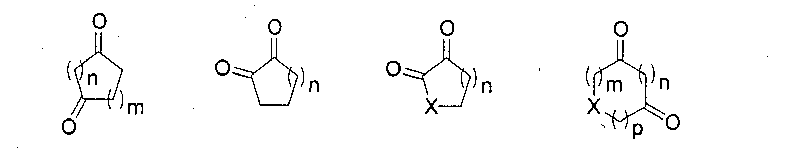

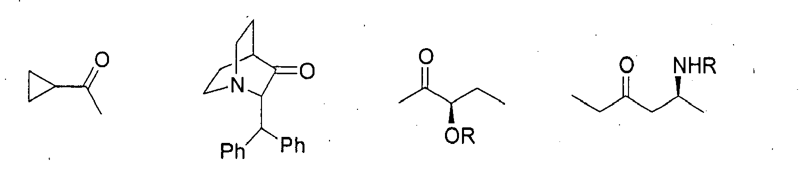

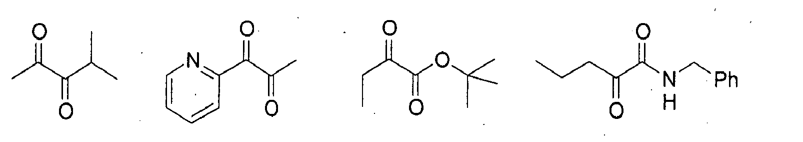

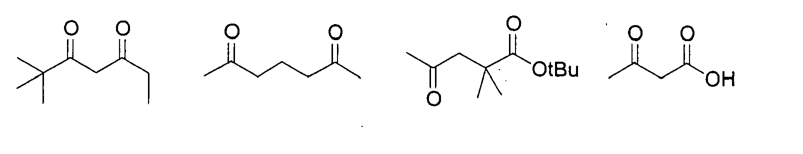

Where any moiety (substituent) R can be connected to another or can be independent from any other moiety R alkyl, alkenyl, alkynyl, aryl, heteroalkyl, heteroalkenyl, heteroalkynyl, heteroaryl, alkylaryl, alkenylaryl, alkynylaryl, heteroalkylaryl, heteroalkenylaryl, heteroalkynylaryl, arylalkyl, arylalkenyl, arylalkynyl, heteroarylalkyl, heteroarylalkenyl, heteroarylalkynyl any -CH2- can be replaced with -CHR- or -CRR- (where any R is as described just before), where m may be any positive integer from 0 onwards, and where n may be any positive integer from 0 onwards and where p may be any positive integer from 0 onwards, and where X denotes a heteroatom.

Furthermore, the method of the present invention can be performed without using a solvent, but is preferably performed in a solvent, wherein said solvent is selected from the group consisting of dichloromethane, tetrahydrofuran, toluene, hexane, tert-butylmethyl ether, 1,2-dimethoxyethane, 1,2-dichloroethane, tetrahydrothiophene-1,1-dioxide, 1,3-dioxolane, dimethylsulfoxide, dimethylformamide, diethylcarbonate, ethyl acetate, methanol, ethanol, or a mixture of two or more thereof. Of these, dichloromethane, tetrahydrofuran, toluene, hexane, tert-butylmethyl ether, 1,2-dimethoxyethane or a mixture of two or more of these solvents are particularly preferred as solvents used in the reductive amination step. These solvents have allowed sufficiently fast reaction times coupled with particularly high yields and diastereomeric excess values of the secondary amine diastereomers. Generally, these solvents also provide the described advantages also in the production of tertiary amine diastereomers according to the method of the invention.

The method according to the present invention is preferably performed at a temperature from -10 °C to 50 °C, and a pressure from 0.5 bar to 50 bar, and a reaction time of up to 48 h. Particularly, the method according to the present invention can be performed at a temperature of 15 °C to 30 °C and a pressure of 2 bar to 8 bar, yielding the desired secondary or tertiary amine diastereomer in high yields and superior diastereomeric excess. Generally, the pressure should be increased if the reductive amination reaction takes more than 20 h to consume 95 % of the (prochiral) ketone educt. Preferred reaction times are 5 h to 20 h, particularly preferred is a reaction time of 5 h to 12 h at a reaction temperature of -10 °C to 30°C and a reaction pressure of 0.5 bar to 10 bar. The reaction time is defined herein as the time necessary to consume 95 % of the limiting reagent.

According to the invention, there is also provided a method for producing a primary or secondary amine product, comprising the steps of
a) performing a method according to the invention as describe above to form a secondary or tertiary amine diastereomer, and then
b) cleaving the remainder of the chiral auxiliary from the amine diastereomer of step a) to yield the primary or secondary amine product.

This method according to the present invention can generally be described as a second step following the aforementioned method according to the invention, particularly according to the second teaching of the invention, as exemplified by the following reaction scheme: 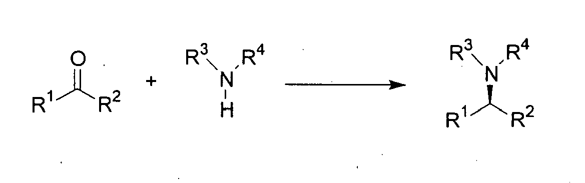 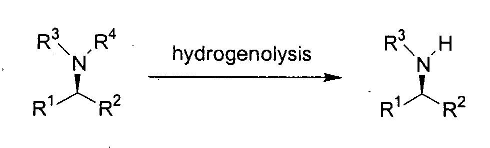 wherein all moieties R1, R2, R3 and R4 have the respective meaning as described above.

The method for producing the primary or secondary amine product according to the invention requires fewer reaction steps than previous methods for producing an enantiopure or enantioenriched primary amine, uses inexpensive and readily available materials, can be performed at ambient temperature and low pressure as described above and provides superior enantiomeric excess at high product yields compared to the other available methods. It exploits the advantages of the method for producing secondary or tertiary amine diastereomers according to the invention as described above.

It is particularly preferred to cleave the remainder of the chiral auxiliary from the respective secondary or tertiary amine diastereomer by hydrogenolysis. A particularly preferred catalyst for use in step b) is one selected from the group consisting of Pd/C and Pd(OH)₂/C, wherein the latter is particularly preferred.

The methods according to the present invention are hereinafter further described by way of examples. It is to be understood that the scope of the invention is defined exclusively by the claims, the examples are not intended to limit the scope of invention.

### Example 1: Synthesis of (R)-4-Phenylbutan-2-amine

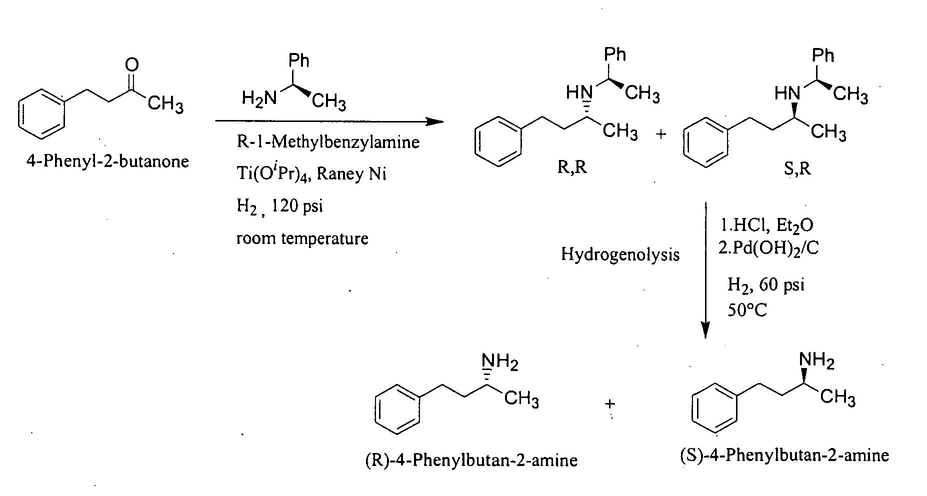

**I. Reductive amination of 4-phenyl-2-butanone.** Under an argon atmosphere, 4-phenyl-2-butanone (1.0 equiv, 0.75 mL, 5.01 mmol) had titanium tetraisopropoxide (1.25 equiv, 1.85 mL, 6.27 mmol) added with stirring followed by R-1-methylbenzylamine (1.1 equiv, 0.71 mL, 5.51 mmol). Raney Nickel (500 mg suspended in toluene (10 mL)), pre-washed first with ethanol twice to remove the water and then with toluene twice , was added to the reaction mixture. The reaction vessel was then pressurized with hydrogen at 120 psi (8.27 bar) at room temperature. The course of the reaction was monitored by gas chromatography. GC analyses were performed on a Shimadzu GC-2010 gas chromatograph using an Rtx-5 amine column (Restec); dimensions: 30 m x 0.25 mm; temperature gradient: 50 °C for 1 min; then 14°C/min up to 280°C, then 5 min at 280 °C; injection temperature: 300°C; detector (FID) temperature 300°C; retention time [min]: minor S,R-isomer: 16.5; major R,R-isomer: 16.7. After 8 h, the reaction was quenched with NaOH (1.0 M, 10 mL) and subsequently stirred for 1 h. The inorganic precipitate along with the catalyst was filtered over a bed of filtering agent, for example celite, the filtering agent was subsequently washed with ethylacetate (4 x 20 mL). The filtrate with the washings were concentrated under reduced pressure and the resultant aqueous suspension was extracted with ethylacetate (2 x 25 mL). The organic extract was dried over Na₂SO₄, filtered and evaporated in vacuum to give the crude product. The diastereomeric products were isolated after column chromatography (cyclohexane/diethylether/NH₄OH = 80:15:05) providing a clear viscous oil (1.14 g, 90% yield), with a diastereomeric ratio of 88:12. The diastereomeric ratio increases to 90:10 (diastereomeric excess = 80%), when the hydrogenation is carried out in dichloromethane. The diastereomers were then separated using gradient column chromatography (cyclohexane/diethyl ether/NH₄OH, 87:08:05 to 80:15:05).

(2R)-4-phenyl-N-((R)-1-phenylethyl)butan-2-amine: R_{f}= 0.41 (cyclohexane:diethyl ether:NH₄OH = 55:40:05); ¹H NMR (400 MHz, CDCl₃) δ 7.32 - 7.16 (m, 10H), 3.87 (q, J = 6.4 Hz, 1H), 2.66 (m, 1H), 2.55 (m, 2H), 1.82 (m, 1H), 1.60 (m, 1H), 1.31 (d, J = 6.4 Hz, 3H), 1.03 (d, J = 6.4 Hz, 3H); ¹³C NMR (100MHz, CDCl₃) δ 146.4, 142.7, 128.5, 128.4, 126.8, 126.6, 125.7, 55.1, 49.8, 38.1, 32.2, 24.6, 21.3; IR (KBr, cm⁻¹ ) 3441; MS (EI, *m*/*z,* %) 253 (M⁺, 5), 238 (16), 148 (78), 105 (100), 44 (42); HRMS (EI) *m*/*z* calcd for C₁₈H₂₃N 253.183, found 253.183.

(2S)-4-phenyl-N-((R)-1-phenylethyl)butan-2-amine: R_{f}= 0.48 (cyclohexane:diethyl ether:NH₄OH = 55:40:05); ¹H NMR (400 MHz, CDCl₃) δ 7.34 - 7.07 (m, 10H), 3.91 (q, J = 6.4 Hz, 1 H), 2.65 (m, 1 H), 2.50 (m, 2H), 1.62 (m, 2H), 1.34 (d, J = 6.4 Hz, 3H), 1.06 (d, J = 5.95 Hz, 3H); ¹³C NMR (100MHz, CDCl₃) δ 146.1, 142.6, 128.5, 128.4, 128.3, 126.8, 126.7, 125.7, 55.0, 49.7, 39.9, 32.5, 25.2, 20.3; IR (KBr, cm⁻¹) 3445; MS (EI, *m*/*z,* %) 253 (M⁺, 8), 238 (20), 148 (78), 105 (100), 44 (40); HRMS (EI) *m*/*z* calcd for C₁₈H₂₃N 253.183, found 253.183.

**II. Hydrogenolysis (cleavage of the chiral auxiliary).** To the mixture of diastereomers (1.01 g, 4.00 mmol) was added ethereal HCl (1.0 M, 5.0 mL) with stirring. The resultant thick suspension was evaporated to give a white solid (1.16 g, 100%). A smaller quantity of the hydrochloride salt (290 mg, 1.00 mmol) was dissolved in methanol (5.0 mL) and hydrogenated in the presence of 20 wt% Pd(OH)₂/C (350 mg, 50 mol%) under a pressure of 60 psi (4.14 bar) at 50°C. Note that an equally effective method (using the same stoichiometry of reagents) involves simple addition of concentrated HCl to a methanolic solution of the diastereomers followed by direct hydrogenation, thereby avoiding the amine salt isolation step. Yields and quality of the product are the same. After 7 h, the catalyst was filtered off and the filtering agent, celite, was washed repeatedly with MeOH. The filtrate along with the washings was evaporated providing a white solid. A 5.0 mL aqueous solution of the white solid at pH 13-14 was then extracted with CH₂Cl₂ (3 x 20 mL). The CH₂Cl₂ extract was dried over Na₂SO₄ and evaporated to give a clear oil (135 mg, 90% yield), which is a mixture of (R) and (S)-4-Phenylbutan-2-amine. The enantiomeric excess of the trifluoroacetylamide derivative of the primary amine product is exactly the same as the diastereomeric excess of the starting diastereomers. The trifluoroacetylamide derivative was prepared in the following manner: To 10 mg of the hydrogenolyzed product (primary amine) in CH₂Cl₂ (5.0 mL) was added trifluoroacetic anhydride (0.4 mL). This was heated at 60°C for 20 min. To the resulting solid residue was added CH₂Cl₂ (5.0 mL), this was washed with aqueous NaHCO₃ (10 mL, 10 weight %/volume). The CH₂Cl₂ extract was passed through a short column of anhydrous Na₂SO₄ (packed in a Pasteur pipette) to remove the residual water. The enantiomeric excess of the sample was determined by chiral GC using a Chiraldex B-DP column (Astec); dimensions: 30 m x 0.25 mm; 150°C, isothermal; carrier gas He @ 23.6 psi; injection temperature 200 °C and detector (FID) temperature 200°C; retention time [min] : Minor component (S)-4-Phenylbutan-2-amine: 4.2 and the major component (R)-4-Phenylbutan-2-amine: 8.1.

### Example 2: Synthesis of (2R)-N-((R)-1-phenylethyl)octan-2-amine

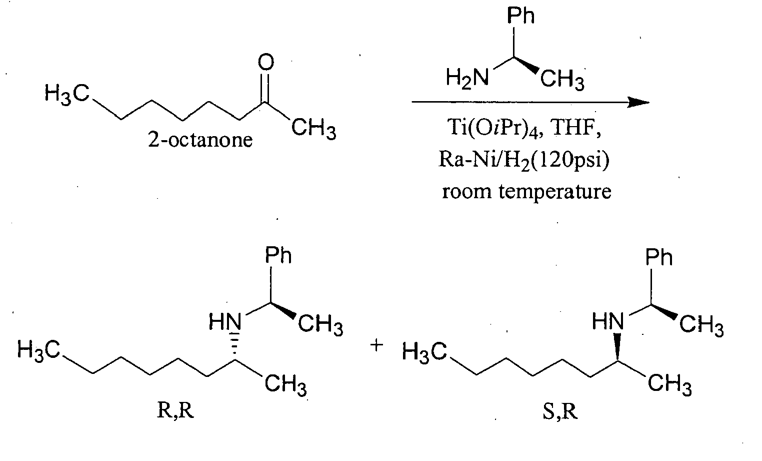

A mixture of 2-octanone (1.0 equiv, 2.49 mmol, 0.39 mL), titanium(IV) isopropoxide (1.25 equiv, 3.13 mmol, 0.92'mL) and (R)-phenylethylamine (1.15 equiv, 2.88 mmol, 0.37 mL) were combined in dry THF (5.0 mL). Simultaneously, the commercial Raney-nickel (400 mg) in water (Fluka-83440) was first added to a round bottom flask and subsequently washed with ethanol two times and then with THF three times under Argon. The resulting final suspension of Raney-nickel was then added to the reaction and the mixture was hydrogenated at 120 psi (8.27 bars) under stirring at room temperature. The reaction was monitered by gas chromatography analysis using a Rtx-5 amine (Restec) column, 0.25 mm x 30 m; temperature gradient: 50 °C for 1 min; then 20 °C/min to 280 °C, then 2 min at 280 °C; injection port temperature: 300 °C; detector (FID) temperature: 300 °C; retention time [min]: minor isomer, 11.0; major isomer, 11.13. At complete conversion, after 6h, the reaction mixture was treated with an aqueous solution of 1.0 M sodium hydroxide (10 mL) and dichloromethane (20 mL) and stirred for 90 min, the solution was then filtered through a filtering agent, celite, and the filtering agent was washed with dichloromethane (3 x 20 mL). Using a separatory funnel, the organic layer was removed and dried over Na₂SO₄, filtered and evaporated to dryness under reduced pressure. Purification by silica gel column chromatography (eluent: diethyl ether/cyclohexane/aqueous ammonium hydroxide, 16:40:1) provided 420 mg (73% yield, 72% diastereomeric excess by GC analysis) of the diastereomers as a pale yellow free flowing liquid. The major diastereomer was isolated after further chromatography, its spectral data are:

**(2R)-N-((R)-1-phenylethyl)octan-2-amine** (R_{*f*} 0.54): IR (KBr) 3440, 1600, 1495, 1448, 1369, 1152, 1113, 756, 704 cm⁻¹; ¹H NMR (400 MHz, CDCl₃) δ 0.88 (t, *J* = 6.4 Hz, 3H), 0.95 (d, J = 6.4 Hz, 3H), 1.25 (brs, 10H), 1.32 (d, J = 6.8 Hz, 3H), 2.49 (m, 1 H), 3.88 (q, J = 6.4Hz, 1H), 7.22-7.34 (m, 5H); ¹³C NMR (100 MHz, CDCl₃) δ 14.1, 21.3, 22.6, 24.6, 25.6, 29.5, 31.8, 36.3, 50.1, 55.1, 126.4, 128.3, 146.5; MS (EI, m/z, %) 233 (M⁺, 5), 218 (85), 148 (84), 106 (16), 105(100), 44 (42); HRMS (EI) calcd for C₁₆H₂₇N (MW) 233.21436, found m/z 233.21385 (M⁺).

### Example 3: Synthesis of (2R)-3-Methyl-N-((R)-1-phenylethyl) butan-2-amine:

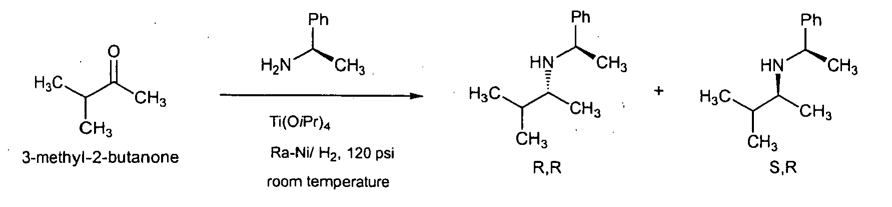

A mixture of 3-methyl-2-butanone (1.0 equiv, 5.00 mmol, 0.53 mL), titanium tetraisopropoxide (1.50 equiv, 7.48 mmol, 2.21 mL), and R-α-methylbenzylamine (1.10 equiv, 5.29 mmol, 0.71 mL) in dry hexane (10 mL) was hydrogenated at 120 psi (8.27 bar) with Raney-Nickel (500 mg) under stirring at room temperature. The aforementioned Raney-Nickel was prepared for the reaction by first adding Raney Nickel in water (Fluka-83440) into a round bottom flask, this was then washed with ethanol twice and then with hexane three times. The Raney-Nickel suspension in hexane was then added to the reaction. The reaction course was monitored by gas chromatography analysis using a Rtx-5 amine (Restec) column, 30 m x 0.25 mm; temperature gradient: 50 °C for 1 min, then 14°C/min to 280 °C, then 5 min at 280 °C; injection port temperature: 300 °C, detector (FID) temperature 300 °C, retention time [min]: major isomer 11.4, minor isomer 11.2. At complete conversion, after 10 h, the reaction mixture was treated with an aqueous solution of sodium hydroxide (1.0 M, 10 mL). After stirring for 1 h, the solution was filtered through a filtering agent, celite, and the celite was then washed with chloroform (2 x 25 mL). In a separatory funnel the organic layer was separated and aqueous layer further extracted with chloroform (20 mL). The combined organic extracts were then dried over sodium sulphate, filtered and finally evaporated to dryness under reduced pressure. Purification by silica gel chromatography (eluent: EtOAc/NH₄OH/Heptane, 4.5:5:90.5) afforded 710 mg (75% yield, 98% diastereomeric excess by GC analysis) of the diastereomers as a clear free flowing oil. The diastereomers were then separated by careful column chromatography (ethylacetate/aqueous ammonium hydroxide/heptane, 3.5:5:91.5).

**(2R)-3-Methyl-N-((R)-1-phenylethyl)butan-2-amine (major isomer):** R_{f} = 0.60 (EtOAc/NH₄OH/Heptane, 10:5:85). ¹H NMR (400 MHz, CDCl₃): δ 7.20-7.31 (m, 5H), 3.85 (q, J = 6.8 Hz, 1 H), 2.41 (m, 1 H), 1.77 (m, 1 H), 1.30 (d, *J* = 6.8 Hz, 3H), 0.82-0.86 (m, 9H). ¹³C NMR (100 MHz, CDCl₃): δ 146.7, 128.2, 126.6, 126.5, 55.1, 54.9, 31.0, 24.6, 19.5, 16.4, 16.3. IR (KBr, cm⁻1): 3424. MS (EI, m/z, %): 191 (M⁺, 5), 176 (50), 148 (58), 105 (100), 44 (40). El HRMS: m/z calcd for C₁₃H₂₁N (M^{+•}) 191.16740, found 191.16791.

**(2S)-3-Methyl-N-((R)-1-phenylethyl)butan-2-amine (minor isomer):** R_{f} = 0.52 (EtOAc/NH₄OH/Heptane, 10:5:85). ¹H NMR (400 MHz, CDCl₃): δ 7.20-7.31 (m, 5H), 3.88 (q, *J* = 6.8 Hz, 1 H), 2.20 (m, 1 H), 1.53 (m, 1 H), 1.32 (d, *J* = 6.8 Hz, 3H), 0.94 (d, *J* = 6.4 Hz, 3H), 0.80-0.85 (m, 6H). ¹³C NMR (100 MHz, CDCl₃): δ 146.2, 128.2, 126.7, 126.6, 54.9, 54.7, 33.3, 25.2, 19.0, 18.0, 16.0. IR (KBr, cm⁻¹): 3432. EI HRMS *m*/*z* calcd for C₁₃H₂₁N [M-15] ⁺ 176.14392, found 176.14392.

### Examples 4-6:

Further experiments were conducted as described in the table below. Reaction parameters were those of examples 1 to 3, that is, according to the second, preferred teaching of the invention. The chiral amine auxiliary used was R-α-methylbenzylamine; the opposite diastereomers were obtained by using S-α-methylbenzylamine. As a mild Lewis acid, titanium (IV) isopropoxide was used. Similar diastereoselectivities (80% and greater) were obtained when aluminium isopropoxide, zirconium isopropoxide, trimethylborate, triisopropylborate, and boron trifluoride were used as mild Lewis acid.

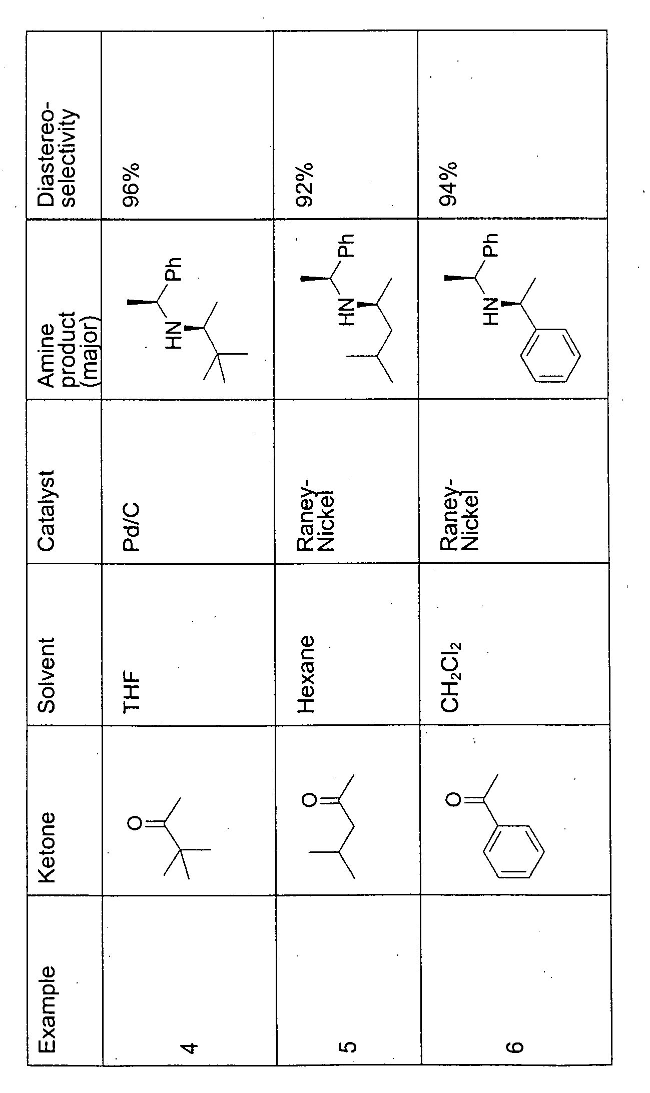

## Claims

1. Method for producing a secondary or tertiary amine diastereomer, comprising or consisting of the step of reductively aminating a ketone with a primary or secondary chiral amine auxiliary to produce the respective secondary or tertiary amine diastereomer, wherein
- the reductive amination is effected in the presence of a hydride,
- optionally with the removal of water, and wherein
- the reductive amination is performed under the influence of a mild Lewis acid.

2. Method for producing a secondary or tertiary amine diastereomer, comprising or consisting of the step of reductively aminating a ketone with a primary or secondary chiral amine auxiliary to produce the respective secondary or tertiary amine diastereomer, wherein
- the reductive amination is effected in the presence of a hydrogenating catalyst and a hydrogenating agent,
- optionally with the removal of water, and wherein
- the hydrogenating catalyst is a metal catalyst selected from the group consisting of nickel, copper, iron, cobalt, ruthenium, rhodium, palladium, osmium, iridium and platinum metal catalysts or mixtures thereof, and wherein
- the reductive amination is performed under the influence of a mild Lewis acid.

3. Method according to any of claims 1 to 2, wherein the mild Lewis acid is selected from the group consisting of a metal alkoxide, a metal acetate, a metal halogenide, a metal amide, a silane, a borate, a heteropolar compound, or a mixture of two or more members of the group.

4. Method according to any of claims 1 to 3, wherein the mild Lewis acid comprises titanium, aluminium, zirconium and/or boron.

5. Method according to any of claims 1 to 4, wherein the metal catalyst is a Raney catalyst, in particular a Raney nickel metal catalyst.

6. Method according to any of claims 1 to 5, wherein the chiral amine auxiliary is present in the reaction in an enantiomeric excess (ee) of ≥70 %.

7. Method according to any of claims 1 to 6, wherein the chiral amine auxiliary is (R)-1-methylbenzylamine or (S)-1-methylbenzylamine.

8. Method according to any of claims 1 to 7, wherein the ketone is of the form 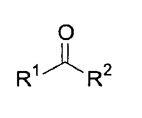 wherein moieties R1 and R2 are, independent from one another,
a) alkyl, alkenyl, alkynyl, aryl, heteroalkyl, heteroalkenyl, heteroalkynyl, heteroaryl, alkylaryl, alkenylaryl, alkynylaryl, heteroalkylaryl, heteroalkenylaryl, heteroalkynylaryl, arylalkyl, arylalkenyl, arylalkynyl, heteroarylalkyl, heteroarylalkenyl, heteroarylalkynyl, and wherein
b) moieties R1 and R2 may together form a ring.

9. Method according to any of claims 1 to 8, wherein the reaction is performed in a solvent, wherein said solvent is selected from the group consisting of dichloromethane, tetrahydrofuran, toluene, hexane, tert-butylmethyl ether, 1,2-dimethoxyethane, 1,2-dichloroethane, tetrahydrothiophene-1,1-dioxide, 1,3-dioxolane, dimethylsulfoxide, dimethylformamide, diethylcarbonate, ethyl acetate, methanol, ethanol, or a mixture of two or more thereof.

10. Method according to any of claims 1 to 9, wherein the reaction is performed at a temperature from -10 °C to 50 °C and a pressure from 0.5 bar to 10 bar and a reaction time of up to 48 h.

11. Method for producing a primary or secondary chiral amine product, comprising the steps of
a) performing a method according to any of claims 1 to 10 to form a respective secondary or tertiary amine diastereomer, and then
b) cleaving the remainder of the chiral auxiliary from the amine diastereomer of step a) to yield the primary or secondary chiral amine product.
